# EUROPEAN PATENT APPLICATION

(11) **EP 2 259 056 A1**
(43) Date of publication of application: **08.12.2010**
(21) Application number: 10009136.2
(22) Date of filing: 15.04.2009
(51) Int. Cl.: G01N 31/22, G01N 21/78

(54) **Apparatus for determining hexavalent chromium**

(30) Priority: 19.05.2008 JP 2008131063
(62) Divisional of application: 09005374.5
(71) Applicant: Hitachi High-Technologies Corporation, Minato-ku Tokyo 105-8717 (JP)
(72) Inventor: Yamamoto, Kazuko, Hitachinaka-shi Ibaraki 312-8504 (JP); Wakui, Takayuki, Hitachinaka-shi Ibaraki 312-8504 (JP); Shirasaki, Toshihiro, Hitachinaka-shi Ibaraki 312-8504 (JP)
(74) Representative: Strehl Schübel-Hopf & Partner

(57) **Abstract**

It is an object of the present invention to provide an analytical method and analyzer for hexavalent chrome that allows for measuring hexavalent chrome conveniently without using a spectrophotometer and obtaining the objective quantification results. Disclosed herein is an analytical method for quantifying hexavalent chrome by, after adding a reagent for complex formation to a liquid sample to form a colored chelate of hexavalent chrome, then contacting the liquid sample with an adsorbent (22), and measuring a thickness of a colored layer of the adsorbent (22) by using a scale (25) on a transparent or translucent column (21).

## Description

### BACKGROUND OF THE INVENTION

### Field of the Invention

The present invention relates to an analytical method and analyzer for easily detecting and quantifying hexavalent chrome contained in a liquid sample such as water.

### Background Art

Hexavalent chrome contained in a liquid sample such as wastewater reacts with a reagent for complex formation such as diphenylcarbazide to form a colored chelate (purple-red chrome-diphenylcarbazone chelate). A method using this for measuring the concentration of hexavalent chrome contained in a liquid sample by a spectrophotometric determination is defined in JIS K0102 65.2.1.

Further, as a conventional technique, JP Patent Publication (Kokai) No. 2007-327886A discloses a method for quantifying hexavalent chrome in water, **characterized in that** a detection material in the powder particle state obtained by complexing silica particles with diphenylcarbazide is contacted with an aqueous sample, and then the concentration of hexavalent chrome is read from changes in color tones of the detection material.

Furthermore, JP Patent Publication (Kokai) No. 63-100357A (1988) discloses a water quality monitoring sensor according to an Ion-Exchanger-Colorimetry, which detects the water quality of, for example sea, river and lake, on site, wherein the color concentration of ion-exchanger which adsorbs a sample containing a metal ion such as Cr and a color reagent such as diphenylcarbazide is optically detected.

Both of the above conventional techniques require a spectrophotometer to detect the resulting colored chelate by absorbance, which made the analyzer costly, and made it difficult to perform measurement immediately at the site where a liquid sample was collected. Meanwhile, there has been known a simple analytical kit to determine the concentration of hexavalent chrome by comparing the resulting colored solution with a color chart without using a spectrophotometer. However, since the color is visually compared, the less objectivity of quantification results has been a problem.

### SUMMARY OF THE INVENTION

As described above, the conventional diphenylcarbazide spectrophotometric determination can quantify the concentration of hexavalent chrome in a liquid sample, but a spectrophotometer is required separately to measure the absorbance. Thus, there has been a problem that the device becomes complicated and costly. Another problem is a poor objectivity of measurements in the case of using a simple analytical kit.

In light of such conventional techniques, it is an object of the present invention to provide an analytical method and analyzer for hexavalent chrome that allows for measuring hexavalent chrome conveniently without using a spectrophotometer and obtaining the objective quantification results.

For the problems described above, the present inventors have found that when adding a reagent for complex formation to a liquid sample to form a colored chelate, and then pouring the colored solution into a column, the thickness of a colored layer of an adsorbent in the column by capturing the colored chelate, is proportionate to the concentration of hexavalent chrome in the liquid sample, thereby being accomplished the present invention.

That is, the summary of the present invention is as follows.
(1) An analytical method for quantifying hexavalent chrome in a liquid sample, comprising adding of a reagent for complex formation to a liquid sample to form a colored chelate of hexavalent chrome, then contacting the liquid sample with an adsorbent, and measuring a thickness of a colored layer of the adsorbent.
(2) The analytical method according to (1) described above, wherein the reagent for complex formation is diphenylcarbazide.
(3) The analytical method according to (1) or (2) described above, further comprising, prior to analysis of a liquid sample, using a standard having a known concentration of hexavalent chrome to determine a correlation between a concentration of hexavalent chrome and a thickness of a colored layer of the adsorbent.
(4) The analytical method according to any one of (1) to (3) described above, further comprising, when the liquid sample is colored, as a pretreatment, the liquid sample is contacted with an adsorbent to remove colored substances before a reagent for complex formation is added thereto to form a colored chelate of hexavalent chrome.
(5) A transparent or translucent column used for the analytical method according to any one of (1) to (4) described above, which is filled with an adsorbent, and provided with a scale on the side, thereby allowing a thickness of a colored layer of the adsorbent to be read from outside.
(6) The column according to (5) described above, which is filled with a mixture of the adsorbent and a glass wool.
(7) The column according to (5) or (6) described above, which is filled with an organic solvent, and allows for eliminating conditioning of adsorbent during analysis.
(8) An analyzer used for the analytical method according to any one of (1) to (4) described above, comprising a column, which is filled with an adsorbent and allows a thickness of a colored layer of the adsorbent to be observed from outside, and an optical sensor system that automatically measures a thickness of a colored layer of the adsorbent.

The present invention allows for quantifying the concentration of hexavalent chrome contained in a liquid sample without using a costly analyzer such as spectrophotometer. Further, since a spectrophotometer is not required, measurement can be performed immediately after collecting a liquid sample at the site where the sample is collected.

Furthermore, by providing a scale on the side of column, it is possible to obtain more objective value than that obtained by a simple analytical method where the concentration is measured by comparing a shade of color with a color chart.

### BRIEF DESCRIPTION OF THE DRAWINGS

FIG. 1 is a drawing illustrating each step of an analytical method for hexavalent chrome according to the present invention.
FIG. 2 is a view showing an embodiment of column.
FIG. 3 is a structural formula showing a representative example of functional group of resin that captures a chelate.
FIG. 4 is a view showing a configuration of an embodiment of an analyzer for hexavalent chrome according to the present invention.

### DESCRIPTION OF SYMBOLS

- 21: Column
- 22: Adsorbent
- 23: Inlet of liquid sample
- 24: Front filter
- 25: Scale
- 26: Back filter
- 27: Outlet of liquid sample

### DETAILED DESCRIPTION OF THE PREFERRED EMBODIMENTS

The present invention will now be described in detail.

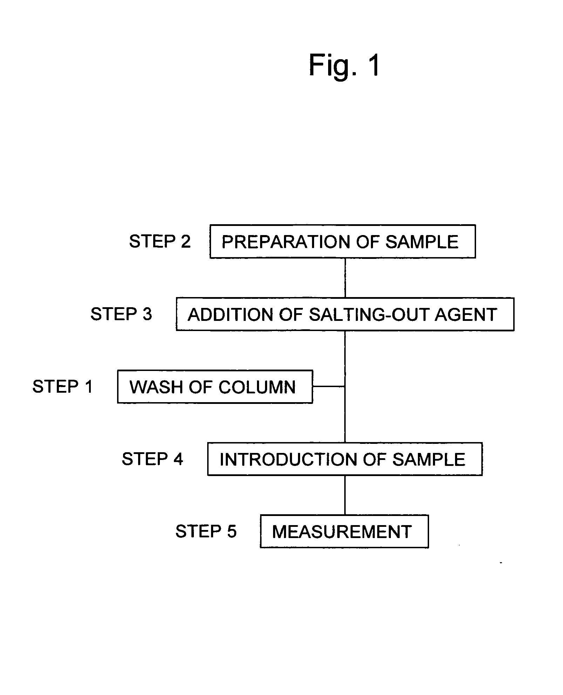

The analytical method for hexavalent chrome according to the present invention is to quantify hexavalent chrome in a liquid sample by, after adding a reagent for complex formation to a liquid sample to form a colored chelate of hexavalent chrome, then contacting the liquid sample with an adsorbent, and measuring a thickness of a colored layer of the adsorbent. Each step during analysis will now be described particularly with reference to FIG. 1.

### Step 1: Wash of column

Firstly, a column used in analysis is prepared and washed. As an external cylinder for the column, a common glass or resin external cylinder can be used, and transparent or translucent one is preferably used to read a thickness of a colored layer of the adsorbent from outside.

The size of the external cylinder of column can be set accordingly with consideration of the quantity of liquid sample to be analyzed, the types of adsorbent, etc. For example, a column having 6 to 30 mm in inner diameter and 50 to 200 mm in length can be employed as a column for analyzing 5 to 50 ml of plating wastewater. A sample with high chrome concentration can be treated by increasing the inner diameter and the like, while quantification of a trace amount of hexavalent chrome can be achieved by reducing the foregoing. Therefore, they are set accordingly as needed.

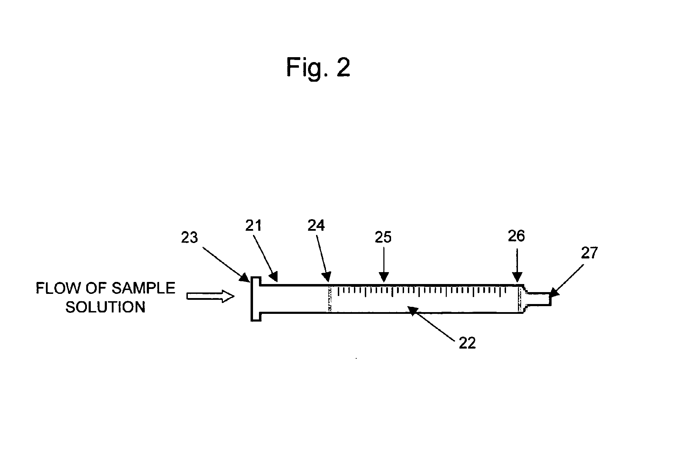

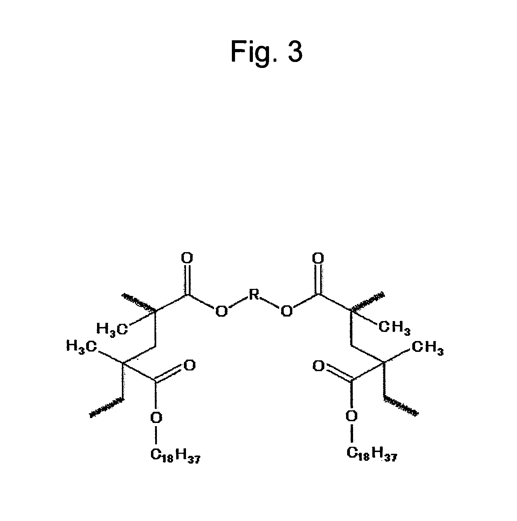

As shown in FIG. 2, the column is produced by providing a front filter 24 and a back filter 26, between which are filled with a given amount of adsorbent 22. The adsorbent to be filled is not particularly limited as long as it captures a chelate of hexavalent chrome. Specific examples of adsorbent include silica fillers such as high porous silica gel, alkyl group-binding silica gel (octylsilylated silica gel, octadecylsilylated silica gel); alumina fillers; and polymer fillers such as polystyrene gel. In particular, an acrylic resin, wherein functional groups (C₁₈) are bonded to oxygen of carboxyl group in methacrylate, or an ODS column, wherein a C₁₈ octadecyl group is bonded to silica gel, as shown in FIG. 3, are preferably used. In addition, the particle size of adsorbent is not particularly limited, and those having the average particle size of 50 to 200 µm can be used.

To optimize the permeability of column to liquid and enhance the accuracy of measurement, adsorbent and other material such as glass wool can be filled in the mixed state. The mixing ratio of adsorbent and other material varies depending on the types of adsorbent or the concentration of hexavalent chrome in a sample, but it is preferable to mix, for example, an acrylic resin and a glass wool in a weight ratio of 2:1 to 1:2.

Further, as shown in FIG. 2, it is preferable to provide a scale 25 on the side of column. This scale can be utilized to accurately read the thickness of colored layer of the adsorbent from outside. The scale of column may be provided with numeral indications by performing a pretest using a standard in advance so that the concentration of hexavalent chrome is directly recognized.

When a column is filled with an organic solvent in advance, a process for conditioning an adsorbent during analysis at the sample-collecting site can be eliminated. As an organic solvent, those conventionally used for conditioning can be applied, and specific example of solvent can include alcohols such as methanol.

A few milliliter of water or the like without containing hexavalent chrome is introduced into the column as described above, followed by flushing out the organic solvent in the column to wash.

### Step 2: Preparation of liquid sample

Subsequently, a reagent for complex formation is added to a liquid sample to form a colored chelate of hexavalent chrome. The liquid sample is not particularly limited as long as it is suspected to contain hexavalent chrome such as wastewater or plating solution. Any reagent for complex formation, which reacts with hexavalent chrome in a sample to form a colored chelate, can be applied. In general, diphenylcarbazide is used. A 1% sulfuric acid solution of diphenylcarbazide defined in JIS may be used as a diphenylcarbazide reagent, but time for reagents preparation or measurement can be saved by using a simple reagent, for example, a reagent set for water analyzer-hexavalent chrome for hexavalent chrome, produced by Kyoritsu Chemical-Check Lab., Corp.

In the case of analyzing an aqueous solution as a liquid sample, which is prepared by treating a chromate coating film such as color screw with boiling water, and then eluting hexavalent chrome, dyes are eluted in a sample and captured by an adsorbent, which hinders the detection of hexavalent chrome. In such case, it is preferred that, as a pretreatment, colored substances such as dyes are removed by contacting a liquid sample colored by dyes with an adsorbent. The types of adsorbent can be selected accordingly depending on the types of colored substances or the like to be removed.

### Step 3: Addition of salting-out agent

A salting-out agent such as sodium chloride is preferably added to a liquid sample, which allows for promoting the adsorption of colored chelate and preventing the diffusion of chelate in an adsorbent layer. The additive amount of salting-out agent may be about 10% by weight of liquid sample. Other than salting-out agent, additives such as pH adjuster may also be added as needed.

### Step 4: Introduction of liquid sample

Then, the liquid sample obtained in Step 3 is poured into a column to contact with an adsorbent. This step allows the adsorbent to capture a colored chelate of hexavalent chrome, which results in coloring of a certain portion of layer thickness. When pouring a liquid sample, all such methods as natural flow-down, pressurized flow-down, and pump suction can be applied. A liquid sample is preferably poured at a velocity of less than 10 ml per minute so that the diffusion of chelate captured by an adsorbent can be prevented.

### Step 5: Measurement

When a liquid sample is poured, each chelate is captured from the end face of adsorbent layer in a column in sequence, resulting in the color of colored layer being changed. By reading the thickness of such colored layer of the adsorbent from the scale, the concentration of hexavalent chrome, which corresponds to a read value, can be identified. It is to be noted that, prior to analysis of liquid sample, a correlation between the concentration of hexavalent chrome and the thickness of colored layer of the adsorbent is preferably determined using a standard having a known concentration of hexavalent chrome.

The layer thickness can be read visually, but a detector that automatically measures the layer thickness of colored portion may be used. For example, an optical sensor system can be employed as such detector. Specifically, the layer thickness of colored portion can be determined by illuminating an adsorbent layer with spot light while moving, for example a LED light source, along with the side of column at a certain speed, detecting reflected light from the adsorbent layer by an optical sensor, and processing signals output from the optical sensor.

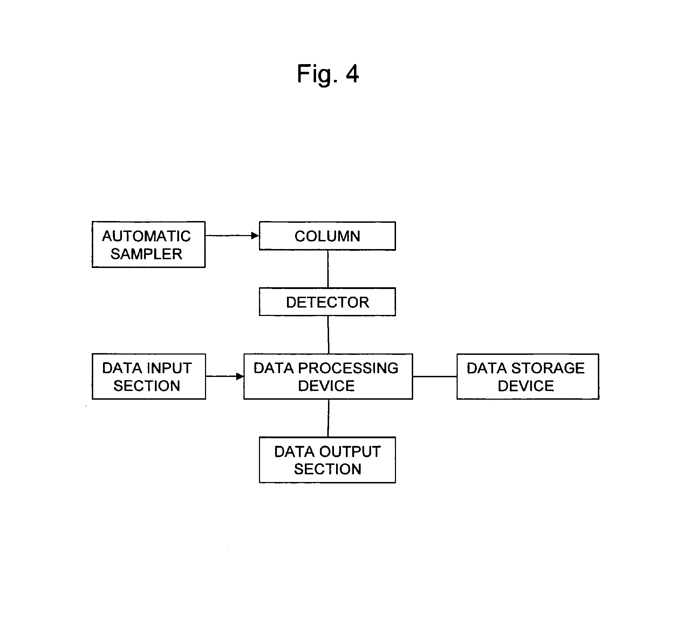

In FIG. 4, an example of device configuration that automatically measures the concentration of hexavalent chrome is shown. In this configuration, a liquid sample is introduced into a column by an automatic sampler; a colored layer of the adsorbent formed in the column is read by a detector such as optical sensor; and then signals are transmitted to a data processing device. In the data processing device, information with regard to the liquid sample, for example the size and name of plated ware (such as color screw), is entered through a data input section and stored in a data storage device, together with the calculated concentration values of hexavalent chrome. The concentration of hexavalent chrome per unit surface area of a chromate coating film of product can be calculated based on both the product size entered through the data input section and the measured concentration value. Data is output from the data output section upon request. The automatic measurement system as shown in FIG. 4 can be used to perform measurements of multiple specimens and data processings.

### Examples

The present invention will now be described in detail with reference to the following examples, but it is not limited thereto.

### Example 1: Wastewater analysis

A column was prepared by filling a glass external cylinder of 12 mm in inner diameter and 50 mm in length, the side of which was calibrated, with an acrylic resin as an adsorbent, which carries a C₁₈ functional group and captures organic substances, to which 3 ml of methanol was poured. In analysis, 5 ml of purified water was poured to wash the column.

Subsequently, a reagent set for water analyzer-hexavalent chrome for hexavalent chrome (diphenylcarbazide), produced by Kyoritsu Chemical-Check Lab., Corp., was added to 25 ml of wastewater as a liquid sample, followed by reaction of Cr (VI) with diphenylcarbazide to form a colored chelate. To the sample containing this colored chelate was added 2.5 g of sodium chloride. This makes it possible to promote the adsorption of chelate to resin and prevent the diffusion of chelate in the adsorbent layer.

Then, the liquid sample containing this colored chelate was poured into the column to contact with the adsorbent. Subsequently, the thickness of the red colored portion of adsorbent layer was read from the scale, and the concentration of Cr (VI) in the liquid sample was measured.

As a liquid sample, wastewater, wherein the concentration of Cr was quantified as 0.5 mg/l by a diphenylcarbazide spectrophotometric determination, was used to perform measurement according to the method described above. As a result, resin was colored near the same scale as when 0.5 mg/l of standard was poured. Accordingly, it was able to confirm that hexavalent chrome was contained in wastewater with the concentration of 0.5 mg/l, which verified a correlation with the value according to the spectrophotometric determination.

### Example 2: Analysis of plating solution

A column was prepared by filling a glass external cylinder of 27 mm in inner diameter and 150 mm in length, the side of which was calibrated, with an acrylic resin as an adsorbent, which carries a C₁₈ functional group and captures organic substances, so as to be 50 mm in height, to which 10 ml of methanol was poured. In analysis, 10 ml of purified water was poured to wash the column.

Subsequently, a liquid sample was prepared by diluting 2.5 ml of plating solution sample with 50 ml of purified water. When this sample was measured by the diphenylcarbazide spectrophotometric determination, the concentration of Cr (VI) was quantified as 78 mg/l. Then, two packages of reagent set for water analyzer-hexavalent chromes for hexavalent chrome (diphenylcarbazide), produced by Kyoritsu Chemical-Check Lab., Corp., were added to this liquid sample, followed by reaction of Cr (VI) with diphenylcarbazide to form a colored chelate. To the sample containing this colored chelate was added 5 g of sodium chloride. This makes it possible to promote the absorption of chelate to resin and prevent the diffusion of chelate in the adsorbent layer.

Then, the liquid sample containing this colored chelate was poured into the column to contact with the adsorbent. Subsequently, the layer thickness of the red colored portion of adsorbent layer was read from the scale, and then the concentration of Cr (VI) in the liquid sample was measured. As a result, it was able to confirm that hexavalent chrome was contained in the diluted plating solution with the concentration of 78 mg/l, which verified a correlation with the value according to the spectrophotometric determination.

### Example 3: Wastewater analysis

A column was prepared by, in 10 ml of methanol, mixing 250 mg of acrylic resin as an adsorbent, which carries a C₁₈ functional group and captures organic substances and 250 mg of silica glass wool, followed by filling a glass external cylinder of 12 mm in inner diameter, the side of which was calibrated, with the resulting mixture so as to be 50 mm in height. In analysis, 5 ml of purified water was poured to wash the column.

Subsequently, a reagent set for water analyzer-hexavalent chrome for hexavalent chrome (diphenylcarbazide), produced by Kyoritsu Chemical-Check Lab., Corp., was added to 25 ml of wastewater as a liquid sample, followed by reaction of Cr (VI) with diphenylcarbazide to form a colored chelate. To the sample containing this colored chelate was added 2.5 g of sodium chloride. This makes it possible to promote the adsorption of chelate to resin and prevent the diffusion of chelate in the adsorbent layer.

Then, the liquid sample containing this colored chelate was poured into the column to contact with an adsorbent. Subsequently, the thickness of the red colored portion of adsorbent layer was read from the scale, and then the concentration of Cr (VI) in the liquid sample was measured. The velocity of liquid sample was 1 ml/min for the column wherein 12 mm of glass external cylinder was filled with 250 mg of resin alone. However, in the case of the column filled with the mixture of 250 mg of resin and 250 mg silica glass, such velocity was 2.5 ml/min. Further, the concentration of Cr (VI) measured according to this example almost matched the value measured according to the spectrophotometric determination.

### Example 4: Analysis of boiling water extract of color screw

To measure the concentration of hexavalent chrome in a chromate coating film of color screw, about 50 cm² of chromate coating film was treated with 50 ml of boiling water, and then hexavalent chrome was eluted. The color screw is stained after formation of chromate coating film. Therefore, dyes are eluted to the boiling water extract, and when the liquid sample is poured into the column, the dyes are captured by an adsorbent together with a colored chelate. As a result, the hexavalent chrome analysis is hindered. Thus, as a pretreatment, dyes were removed by pouring the boiling water extract, to which diphenylcarbazide reagent had yet to be added, into the column filled with resin that captures organic substances.

Subsequently, a reagent set for water analyzer-hexavalent chrome for hexavalent chrome (diphenylcarbazide), produced by Kyoritsu Chemical-Check Lab., Corp., was added to 25 ml of color screw boiling water extract from which dyes were removed, followed by reaction of Cr (VI) with diphenylcarbazide to form a colored chelate. To the sample containing this colored chelate was added 2.5 g of sodium chloride. This makes it possible to promote the adsorption of chelate to resin and prevent the diffusion of chelate in the adsorbent layer.

A column was prepared by filling a glass external cylinder of 12 mm in inner diameter, the side of which was calibrated, with an acrylic resin as an adsorbent, which carries a C₁₈ functional group and captures organic substances so as to be 15 mm in height, to which 3 ml of methanol was poured. In analysis, 5 ml of purified water was poured to wash the column.

Then, the boiling water extract containing the colored chelate was poured into the column to contact with an adsorbent. Subsequently, the thickness of the red colored portion of adsorbent layer was read from the scale, and then the concentration of Cr (VI) in the boiling water extract was measured. The concentration of Cr (VI) thus measured was 0.06 mg/l, which almost matched the value measured according to the diphenylcarbazide spectrophotometric determination.

A method and device according to the present invention can be utilized for wastewater analysis and chrome plating extract, which makes it possible to know the concentration of hexavalent chrome at the site where a sample is collected.

## Claims

1. A transparent or translucent column (21) used for an analytical method for quantifying hexavalent chrome in a liquid sample, the method comprising adding a reagent for complex formation to a liquid sample to form a colored chelate of hexavalent chrome, then contacting the liquid sample with an adsorbent (22), and measuring a thickness of a colored layer of the adsorbent (22), the column (21) being filled with a mixture of the adsorbent (22) and a glass wool, and provided with a scale (25) on the side, thereby allowing a thickness of the colored layer of the adsorbent (22) to be read from outside.

2. An analyzer comprising
the transparent or translucent column (21) of claim 1, and
an optical sensor system for automatically measuring a thickness of the colored layer of the adsorbent (22).
